# EUROPEAN PATENT SPECIFICATION

(11) **EP 1 421 974 B1**
(45) Date of publication and mention of the grant of the patent: **06.12.2006**
(21) Application number: 02425379.1
(22) Date of filing: 10.06.2002
(51) Int. Cl.: A61P 27/04, A61K 31/716

(54) **Carboxy methyl beta-1, 3 glucan containing ophthalmic solution**
Carboxy Methyl Beta-1,3 Glucan enthaltende ophthalmische Lösung
Solution ophthalmique comprenant carboxy methyl beta-1, 3 glucan

(43) Date of publication of application: 26.05.2004
(73) Proprietor: D.M.G. Italia Srl, 00040 Pomezia RM (IT)
(72) Inventor: Mercuri, Luigi, 00181 Roma (IT)
(74) Representative: Sarpi, Maurizio

(56) References cited:
- EP-A- 0 819 703
- WO-A-00/54747
- WO-A-01/37851
- WO-A-90/12564
- WO-A-95/22310
- WO-A-99/41282
- ZÜLLI F ET AL: "CM-Glucan a new yeast polysaccharide for cosmetic use" COSMETICS AND TOILETRIES MANUFACTURE WORLDWIDE, BUSHEY HEATH, GB, 1994, pages 131,133-134,136, XP002090001 ISSN: 1358-2453
- ZULLI F ET AL: "Improving skin function with CM-glucan, a biological response modifier from yeast." INTERNATIONAL JOURNAL OF COSMETIC SCIENCE, vol. 20, no. 2, April 1998 (1998-04), pages 79-86, XP002213159 ISSN: 0142-5463
- PELLEGRINI G ET AL: "Location and Clonal Analysis of Stem Cells and their Differentiated Progeny in the Human Ocular Surface" THE JOURNAL OF CELL BIOLOGY, ROCKEFELLER UNIVERSITY PRESS, US, vol. 145, no. 4, 17 May 1999 (1999-05-17), pages 769-782, XP002192318 ISSN: 0021-9525

## Description

The present invention concerns the field of pharmaceuticals and more particularly it refers to the preparation of an glucan-containing ophthalmic solution, able to: increase the hydration and the lubrication of the cornea, facilitate regeneration processes, protect cornea from the harmful effects of solar radiations and having immunostimulating effects.

As it is well known, the cornea is the lens of the ocular optic system, in facts, it is able to refract light and direct it toward the visual perception structures of the retina and, together with the crystalline, it is responsible for images focusing. The cornea histology and metabolism is responsible for cornea's high transparency maintenance needed to carry out its specific functions.

The cornea has the following histologic structure, wherein three layers can be distinguished:
- a multylayer pavement epithelium lying on the basal membrane;
- a connectival stroma consisting in a matrix of mucopolysaccharides, collagen fibers and keratinocytes;
- a monolayer endothelium having high metabolic activity.

The chemical composition of the cornea is the following: 80% water, 16% proteins, especially collagen, 4% mucopolysaccharides, lipids and mineral salts.

In particular, mucopolysaccharides, representing the main constituent of the connectival matrix, are linear polymeric chains of aminosugars: glycosamine and galactosamine. Mucopolysaccharides can be classified as follows:
- acidic mucopolysaccharides, have a negative charge due to the presence of uronic and sulfate residues (i.e. keratan sulfate, chondroitin and chondroitin sulfate), they are able to fix cations, to bind many water molecules and to help matrix fibers cohesion;
- neutral structural mucopolysaccharides or glycoprotein, i.e. keratoglycosaminglycan (KGAG) which is the main component of the cement that bounds tropocollagen molecules in the matrix.

Beta-(1-3)-Glucans, which are the fundamental components of cellular walls of saprophytic fungi, pathogens and some bacteria, have risen high scientific interest. In fact, it is already known that said molecules are able to activate the host defences because they interact with specific receptors of the plasmatic membrane of immune competent cells as macrophages and monocytes.

In particular, it has been demonstrated that beta - Glucan, from baker's yeast S.Cerevisiae, is able to inhibit human monocyte phagocytosis of particulate antigens, because it binds to the above mentioned receptors causing a decreased availability of the receptor for a subsequent phagocytic response to particulate activators. (Joyce K. CZOP and K. Frank Austen, 1985, The J. of Immunol., 134 (4), 2588-2593). Recently, it has been demonstrated that beta-Glucan can bind to the surface of cells which are not immune competent i.e. NHDF (normal human dermic fibroblast), (Kougias, P., et to the., 2001, Infection and Immunity, 69(6), 3933-3938).

Further effects of beta-(1-3) - Glucan have been demonstrated, as for example, its ability to induce wound healing (WO 90/12564A; EP-A-0 819 703). In fact, its topical or intravenous administration results in wound healing because of its stimulation of macrophages metabolism. Moreover, macrophages themselves induce fibroblast migration and proliferation, synthesis of the collagen and local angiogenesis (Browder W., et al., 1988, Surgery, 104 (2), 224-230).

Many tests has been conducted to evaluate the use of beta-(1-3) - Glucans for the preparation of products for topical administration. Is has been demonstrated that that the insoluble glucans can have undesired toxic effects and consequently a water soluble form of the beta (1-3)Glucan has been identified: Carboxy Methyl beta-glucan (CMG), which can be easily and safely used in pharmaceuticals and cosmetics. In particular, CMG has shown the ability to induce in vitro cellular proliferation and to reduce oxidative stress caused by U.V. rays. Tests conducted in vivo, applying oil-in-water emulsions or hydrogel containing CMG in a rate from 0,04 to 0,4% on patients' skin, have demonstrated that said preparations are able to increase skin humidity, cellular proliferation and tissue renewal and, in addition, to inhibit squalene oxidation (main lipidic component of sebum) and its consequent release of free radicals induced by U.V. rays (Zulli F., et to the., 1996, Cosmetics & Toiletries, 111 (12), 91-96; 2ugli et al., 1994, Cosmetics & Toiletries, 131-136; Zulli et al., 1998, Intl. J. Cosmetic Sci., 20(2), 79-86; WO 00/54747; WO 99/41282; Pellegrini et al., 1999, J. Cell. Biol., 145(4), 769-782; WO 01/37851).

In the specific ophthalmic field, particular interest has been focused, up to now, to the preparation of the so-called "artificial tears", i.e. acqueous solutions able to simulate the function of the lacrimal fluid used to maintain and/or restore the eye natural lubrication. Said preparations, up to now, contains certain types of polysaccharides which are not able to trigger they own synthesis by the corneal keratinocytes.

Further studies conducted on different types of preparations, to be used for the above specified purpose, have shown that ophthalmic solutions, containing water-soluble Carboxy Methyl beta-glucan, have many favourable features in comparison to up to now preparations, containing other types of polysaccharides.

In fact, the experimental tests that led to the definition of the preparation, subject matter of the present invention, underlined that Carboxy Methyl beta-glucan increases directly and indirectly cornea lubrication and hydration. More precisely, CMG is not only a lubricant by itself, but it is able, at the same time, to stimulate corneal keratinocytes proliferation, that are the cells responsible for mucopolysaccharides synthesis. Thus, the capability to induce eye lubrication is extraordinarily enhanced. Beside this unexpected effect supplementary characteristics are present: i.e. immunostimulating and antioxidant activities and stimulation of tissue regeneration due to fibroblasts.

The experiments lead to the following preferred composition of the immune stimulating, regenerating and re-hydrating preparation according to the present invention, in the form of an aqueous solution for ophthalmic use containing 0.2% to 2% Carboxy Methyl beta 1,3-glucan (CMG) more preferably containing 0.5% CMG and, most preferably having the following composition (table I).

**TABLE I**

| | |
|---|---|
| Carboxy Methyl beta 1,3-glucan (CMG) | 0,5% |
| N-Hydroxymethyl glycinate | 0,001% |
| Sodium edetate | 0,1% |
| Isotonic solution (pH 7,2) | q.b. |

The immunostimulating preparation in a dose of 15 ml has the following composition (TABLE II):

**TABLE II**

| | |
|---|---|
| Carboxy Methyl beta 1,3-glucan (CMG) | 80 µl |
| N-Hydroxymethyl glycinate | 0,2 µl |
| Sodium edetate | 15 µl |
| Isotonic solution (pH 7,2) | q.b. |

In the following table III, referred to 100 ml of the product in the aqueous solution form, the range of concentrations (percentage) of the pharmacologically active Carboxy Methyl beta-glucan, is indicated, being its therapeutic effects substantially maintained.

**TABLE III**

| | |
|---|---|
| Carboxy Methyl beta 1,3-glucan (CMG) | 0,2% + 2% |

In conclusion, the preparation, subject matter of the present invention, can be advantageously used to enhance corneal epithelium regeneration processes, to relief corneal hypersensitivity due to traumatic lesions also of post-surgery nature or due to protracted exposure to solar rays or video terminals emitted radiations.

## Claims

1. The use of Carboxy Methyl beta 1,3-glucane for the preparation of a pharmaceutical composition for ophthalmic use to improve indirect ocular lubrication and to promote regenerating process.

2. Pharmaceutical composition for ophthalmic use improving indirect ocular lubrication and promoting regenerating processes wherein it contains Carboxy Methyl beta 1,3-glucan (CMG) in aqueous solution.

3. Pharmaceutical composition for ophthalmic use according to claim 2 containing Carboxy Methyl beta 1,3-glucan (CMG) in aqueous solution in a range from 0,2% to 2%.

4. Pharmaceutical composition for ophthalmic use according to claims 2 and 3 containing 0,5% Carboxy Methyl beta 1,3-glucan.

5. pharmaceutical composition for ophthalmic use for improve indirect ocular lubrication and for promoting regenerative process having the following composition:
| | |
|---|---|
| Carboxy Methyl beta 1,3-glucan (CMG) | 0,5% |
| N-Hydroxymethyl glycinate | 0,001% |
| Sodium edetate | 0,1% |
| Isotonic solution (pH 7,2) | q.b. |

6. Pharmaceutical composition for ophthalmic use according to claims 2, 3, 4 and 5 able to enhance corneal hydration and indirect lubrication.

7. Pharmaceutical composition for ophthalmic use according to claims 2, 3, 4 and 5 having immunostimulating effect.

8. Pharmaceutical composition for ophthalmic use according to the claims 2, 3, 4 and 5 able to induce corneal epithelium regeneration.

9. Pharmaceutical composition for ophthalmic use according to the claims 2, 3, 4 and 5 able to protect cornea from solar radiation.

## Patentansprüche

1. Verwendung von Carboxymethyl-Beta-1,3-Glukan zur Herstellung einer pharmazeutischen Zusammensetzung zur ophthalmischen Verwendung, um die indirekte Okularbenetzung zu verbessern und den Regenerationsprozess zu fördern.

2. Pharmazeutische Zusammensetzung zur ophthalmischen Verwendung, um die indirekte Okularbenetzung zu verbessern und die Regenerationsprozesse zu fördern, wobei die Zusammensetzung Carboxymethyl-Beta-1,3-Glukan (CMG) in Form einer wässrigen Lösung enthält.

3. Pharmazeutische Zusammensetzung zur ophthalmischen Verwendung nach Anspruch 2, enthaltend Carboxymethyl-Beta-1,3-Glukan (CMG) in Form einer wässrigen Lösung in einem Bereich von 0,2% bis 2%.

4. Pharmazeutische Zusammensetzung zur ophthalmischen Verwendung nach einem der Ansprüche 2 und 3, enthaltend 0,5% Carboxymethyl-Beta-1,3-Glukan.

5. Pharmazeutische Zusammensetzung zur ophthalmischen Verwendung zur Verbesserung der indirekten Okularbenetzung und zur Förderung des Regenerationsprozesses mit der nachfolgenden Zusammensetzung:
| | |
|---|---|
| Carboxymethyl-Beta-1,3-Glukan (CMG) | 0,5% |
| N-Hydroxymethylglycinat | 0,001% |
| Natriumedetat | 0,1% |
| Isotonische Lösung (pH 7,2) | q.b. |

6. Pharmazeutische Zusammensetzung zur ophthalmischen Verwendung nach einem der Ansprüche 2, 3, 4 und 5, die in der Lage ist, die Korneahydration und die indirekte Benetzung zu verbessern.

7. Pharmazeutische Zusammensetzung zur ophthalmischen Verwendung nach einem der Ansprüche 2, 3, 4 und 5 mit Immun stimulierender Wirkung.

8. Pharmazeutische Zusammensetzung zur ophthalmischen Verwendung nach einem der Ansprüche 2, 3, 4 und 5, die in der Lage ist, die Korneaepithelregeneration zu enduzieren.

9. Pharmazeutische Zusammensetzung zur ophthalmischen Verwendung nach einem der Ansprüche 2, 3, 4 und 5, die in der Lage ist, die Kornea vor Sonnenstrahlung zu schützen.

## Revendications

1. Utilisation de carboxy-méthyl-béta-1,3-glucane pour la préparation d'une composition pharmaceutique ophtalmique utilisée pour améliorer la lubrification oculaire indirecte et pour favoriser le procédé de régénération.

2. Composition pharmaceutique ophtalmique utilisée pour améliorer la lubrification oculaire indirecte et pour favoriser le procédé de régénération, laquelle contient du carboxy-méthyl-béta-1,3-glucane (CMG) en solution aqueuse.

3. Composition pharmaceutique ophtalmique selon la revendication 2, laquelle contient du carboxy-méthyl-béta-1,3-glucane (CMG) en solution aqueuse dans une gamme comprise entre 0,2% et 2 %.

4. Composition pharmaceutique ophtalmique selon la revendication 2 et 3, contenant 0,5% de carboxy-méthyl-béta-1,3-glucane (CMG).

5. Composition pharmaceutique ophtalmique utilisée pour améliorer la lubrification oculaire indirecte et pour favoriser le procédé de régénération, présentant la composition suivante :
| | |
|---|---|
| Carboxy-méthyl-béta-1,3-glucane (CMG) | 0,5% |
| Glycinate de N-Hydroxyméthyl | 0,001% |
| Edédate de sodium | 0,1% |
| Solution isotonique (pH 7,2) | qsp |

6. Composition pharmaceutique ophtalmique selon la revendication 2, 3, 4 et 5, permettant d'améliorer la lubrification indirecte et l'hydratation de la cornée.

7. Composition pharmaceutique ophtalmique selon la revendication 2, 3, 4 et 5, présentant un effet immunostimulant.

8. Composition pharmaceutique ophtalmique selon la revendication 2, 3, 4 et 5, permettant d'induire une régénération de l'épithélium de la cornée.

9. Composition pharmaceutique ophtalmique selon la revendication 2, 3, 4 et 5, permettant de protéger la cornée des radiations solaires.
